# FASCICULE DE BREVET EUROPEEN

(11) **EP 1 741 411 B1**
(45) Date de publication et mention de la délivrance du brevet: **03.12.2014**
(21) Numéro de dépôt: 06012467.4
(22) Date de dépôt: 17.06.2006
(51) Int. Cl.: A61F 2/36

(54) **Gamme de prothèsesn fémorales de première intention et dispositif de préparation d'un fût fémoral associé.**
Femurprothesenset für den Ersteinsatz und Vorrichtung zum Vorbereiten des Femurs.
Set of femoral prothesis for the first application and device for preparing the femur.

(30) Priorité: 29.06.2005 FR 0506755
(43) Date de publication de la demande: 10.01.2007
(73) Titulaire: Zimmer France SAS, 25461 Etupes Cedex (FR); Le Beguec, Pierre, 35000 Rennes (FR)
(72) Inventeur: Le Beguec, Pierre, 25000 Besancon (FR)
(74) Mandataire: Novagraaf Technologies

(56) Documents cités:
- FR-A- 2 839 878
- FR-A- 2 853 524
- US-A- 5 108 451
- US-A- 5 387 243
- US-A- 5 702 487

## Description

La présente invention concerne une gamme de prothèses fémorales de première intention, c'est-à-dire posée pour la première fois sur un patient. Ces prothèses sont implantées sans ciment dans un fût fémoral, dans une zone dite « Press-Fit ».

Pour assurer la fixation sans micro-mouvement d'une tige fémorale dans le fémur, et assurer ainsi la pérennité du montage, le mode de fixation peut être un ciment acrylique ou une fixation mécanique.

La présente invention se situe dans le cadre d'une prothèse de première intention fixée d'une façon mécanique sans mouvement interstitiel.

Différents concepts de prothèses existent, ayant fait l'objet de nombreuses inventions.

Le premier principe consiste à évider au maximum le fémur et à épouser son anatomie (endomédullaire) en ayant une prothèse qui, par ses courbures, va permettre un contact de surface intime entre l'implant et l'os cortical résiduel (fût fémoral).

La majorité de ces implants ont une forme dite anatomique avec une partie supérieure renflée, ce qui rend difficile l'enfoncement et donc le blocage de ces implants et, dans ces conditions, le contact entre l'os et l'implant n'est pas toujours aussi intime qu'on le souhaiterait.

D'autres types de fixation vont privilégier les prothèses droites qui vont se stabiliser, non pas par un contact de surface entre l'os et l'implant, mais par un appui 3 points, le plus souvent dans un plan sagittal (de profil) au niveau des parties antérieure et postérieure du fémur. Il s'agit de prothèses de type autoblocantes dites « Zweymuller », de section rectangulaire.

Ce mode de stabilisation présente plusieurs inconvénients :
- Un appui 3 points ne permet pas un bon contrôle des contraintes en rotation, ce qui est pourtant essentiel quand on a fait le choix d'une prothèse sans ciment.
- La longueur des prothèses qui se stabilisent par appui 3 points est, par nécessité, relativement importante, en moyenne de 16 cm. De plus, et d'une façon habituelle, plus la taille (diamètre) de l'implant augmente, plus sa longueur totale augmente. Ainsi, si l'on prend pour exemple les tiges « Zweymuller » et pour référence la longueur correspondant à la distance qui sépare l'épaulement de l'extrémité distale, on peut noter une différence de 58 millimètres entre la taille 01 la plus petite et la taille 12 la plus grande.
- La longueur de ces implants a pour effet d'induire un autre inconvénient : plus l'implant est long, moins on a de chance d'obtenir un contact entre l'os et l'implant sous forme d'une surface surtout en zone proximale du fémur qui est pourtant la zone privilégiée pour assurer la stabilité d'un implant sans ciment.
- Enfin, la mise en place d'un implant long nécessite une large vision du canal fémoral car, avec ces implants, l'utilisation des râpes et alésoirs suppose d'être toujours coaxial pour éviter tout risque de fracture ou de fausse route.
   L'invention s'inscrit dans le cadre du concept « Press-Fit » pour assurer une parfaite stabilité primaire d'une tige fémorale sans ciment. Elle s'inscrit également dans le cadre de l'avènement de la chirurgie mini-invasive, en soulignant que ce concept nécessite un matériel ancillaire adapté aux mini voies d'abord et, si l'on veut respecter une certaine logique dans la démarche, la mise en place d'une prothèse également mini-invasive, ce qui, dans le domaine des prothèses fémorales, signifie une tige courte.
   Pour la demanderesse, le concept « Press-Fit » nécessite d'obtenir, dans un premier temps et dans tous les cas, un contact sous forme d'une surface entre l'os fémoral et l'implant, et ceci dans la région proximale du fémur. Pour atteindre cet objectif, le chirurgien est confronté à 3 problèmes qu'il doit solutionner simultanément.
- Il doit faire usage d'une tige courte pour éviter le risque d'un appui 3 points et pour respecter la notion de chirurgie mini-invasive avec une tige également mini-invasive.
- Ensuite, il doit trouver la zone idéale pour assurer un contact os-implant sous forme d'une surface (c'est-à-dire la zone « Press-Fit »), sachant que cette zone se situe à une hauteur variable selon le morphotype et l'importance des défauts osseux (dus par exemple à une ostéoporose). Cette zone se situe rarement en région métaphysaire haute, c'est-à-dire au dessus du petit trochanter, car il y a à ce niveau de nombreuses variations anatomiques, en particulier lorsqu'il s'agit d'une prothèse de première intention. La zone du fémur, qui est en définitive la plus sûre pour assurer régulièrement un contact de surface, se situe en zone métaphysodiaphysaire, c'est-à-dire au niveau et un peu en dessous du petit trochanter, sachant qu'à ce niveau, la zone de stabilité peut se situer à une hauteur qui peut varier de plusieurs millimètres, et sachant aussi qu'il est toujours préférable d'éviter une fixation serrée plus bas, en région diaphysaire.
- Enfin, l'opérateur doit implanter une prothèse dans la zone idéalement préparée tout en restituant la bonne longueur du membre inférieur, ce qui est parfois délicat compte tenu de la variabilité en hauteur de la zone « Press-Fit ».

La présente invention a donc pour but de remédier à l'ensemble des inconvénients précités, et concerne à cet effet une gamme de prothèses fémorales de première intention, à implanter sans ciment dans un fût fémoral, dans une zone dite de « Press-Fit » se situant dans celui-ci à une hauteur variable selon le morphotype et l'importance des défauts osseux.

Cette gamme de prothèses est caractérisée en ce qu'elle comprend, pour chaque diamètre, au moins deux tiges fémorales d'une longueur identique et avec un angle cervico-diaphysaire également identique, mais dont la zone conique utile pour le blocage de l'implant et assurer sa stabilité se situe à une hauteur différente, et sur l'une des tiges, cette zone se situe dans une position plus basse que l'autre. La variation en hauteur du positionnement de la zone conique, dite zone de blocage, et qui correspond à la zone « Press-Fit » du fût fémoral préalablement préparé, permet d'assurer la stabilité primaire de l'implant, tout en respectant l'égalité de longueur des deux membres inférieurs et en implantant à chaque fois une tige la plus courte possible.

L'invention concerne également un système modulable de préparation du fémur, qui permet une préparation étagée de celui-ci, ce qui signifie de trouver et préparer, dans un premier temps, la zone de stabilité primaire dite zone « Press-Fit », et dans un deuxième temps, de préparer le fémur dans sa zone proximale pour choisir la tige fémorale prothétique la mieux adaptée pour restituer la bonne longueur du membre inférieur.

A ce propos, une solution a déjà été proposée dans la demande de brevet d'invention FR-A-2839878 déposée par la demanderesse, mais qui n'était pas initialement destinée à la mise en place de prothèses fémorales selon la présente invention.

### L'invention se résume donc de la manière suivante :

La préparation de l'aire d'implantation d'une prothèse fémorale sans ciment, avec une râpe modulable, permettant d'assurer la stabilité primaire de l'implant sur une distance réduite, ce qui peut également permettre la mise en place d'une prothèse fémorale qui a pour caractéristique avantageuse d'être courte. De plus, en faisant varier le positionnement de la zone conique de l'implant, cette prothèse peut être toujours de la même longueur, quel que soit son diamètre, et il est facile de régler la longueur du membre inférieur.

Trois avantages très importants sont procurés par l'invention et sont à mettre en évidence.
1/ Dans le domaine du sans ciment, l'implantation d'une tige courte est toujours un avantage, ne serait-ce que parce qu'elle sera toujours facile à extraire de son logement le moment venu, surtout si elle n'est pas descellée. Tout implant mis en place doit pouvoir être enlevé sans trop de dommages pour l'os environnant.
2/ Quand la stabilité primaire est assurée par effet « Press-Fit », avec un implant d'une forme conique, il faut impérativement obtenir un contact os-implant sous forme d'une surface, et l'on comprend facilement qu'il est plus aisé, et en définitive plus sûr, de bien préparer une surface de contact os-implant sur une hauteur réduite. Vouloir rendre conique un segment de fémur sur une longue distance (au-delà de 4 à 5 centimètres) est illusoire.
3/ La stabilité primaire par effet « Press-Fit » est de meilleure qualité si elle est assurée sur une hauteur réduite avec un contact os-implant sous forme d'une surface, ce qui est possible grâce à l'invention. Plus une tige est longue, plus la hauteur de la zone de contact os-implant est importante, et plus les risques d'un appui « 3 points » sont importants, et pour une tige sans ciment, ceci n'est jamais souhaitable.

Ensuite, un avantage à utiliser une râpe modulable, qui fait également office de prothèse d'essai, facilite les différentes manoeuvres de réglage nécessaires en cours d'intervention. Dans le cadre de la chirurgie mini-invasive, l'exposition du fémur proximal est par définition réduite et les repères pour apprécier, par exemple la stabilité ou l'orientation de la prothèse, ne sont pas toujours d'accès facile et fiable.

Avec une râpe modulable, il est possible de laisser la partie distale de la râpe en place (sans la partie proximale) pour exécuter, par exemple, un contrôle radiologique en cours d'intervention et s'assurer du bon positionnement de la zone conique d'ancrage, qui doit être en parfait contact avec les corticales.

De la même manière, on peut également faire varier le déport et/ou la longueur du col, voire l'angle cervico-diaphysaire, de la partie proximale de la prothèse d'essai, ce qui, en définitive, permet de choisir en cours d'intervention l'implant définitif monobloc qui restitue le mieux l'architecture de la hanche et la bonne longueur du membre inférieur.

La présente invention concerne également les caractéristiques qui ressortiront au cours de la description qui va suivre, et qui devront être considérées isolément ou selon toutes leurs combinaisons techniques possibles.

Cette description donnée à titre d'exemple non limitatif, fera mieux comprendre comment l'invention peut être réalisée en référence aux dessins annexés sur lesquels:

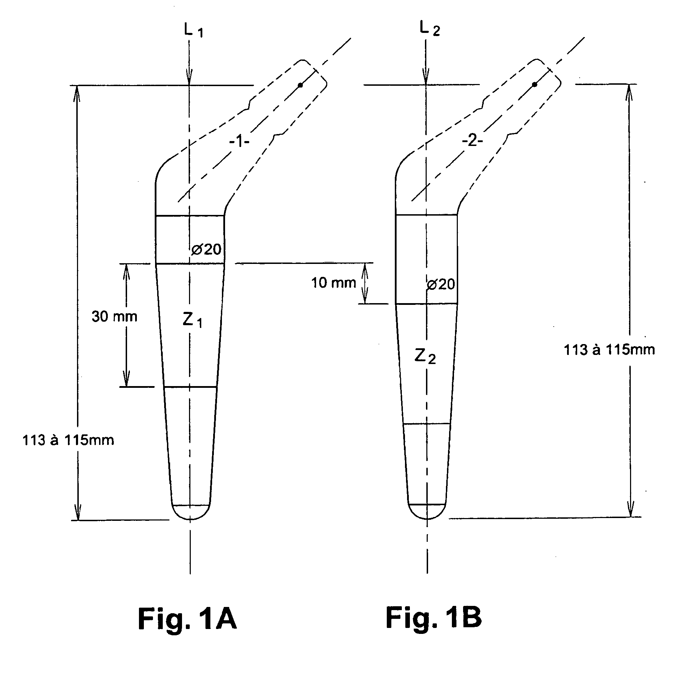

Les figures 1A et 1B représentent une prothèse faisant partie d'une gamme de prothèses fémorales selon l'invention, à savoir un ensemble de tiges courtes et d'une longueur constante quelle que soit la taille du fémur.

C'est ainsi que, pour une taille déterminée, qui, dans cet exemple, est un diamètre de 20 mm, il est possible de réaliser une gamme intermédiaire d'au moins deux prothèses fémorales de première intention qui peuvent être implantées et se stabiliser dans un fémur de la même taille mais d'un morphotype différent. Dans l'exemple pris ici, le nombre des prothèses intermédiaires est de 2, mais ce nombre peut être différent (par exemple 3) sachant toutefois que ce nombre est au minimum de 2.

Toujours selon le présent exemple de réalisation, pour une tige d'un diamètre de 20 mm, la gamme intermédiaire comprend deux tiges fémorales 1, 2 d'une forme conique, de longueurs identiques L1, L2, et avec des angles cervico-diaphysaire également identiques.

La zone conique Z1 ou Z2 destinée à assurer l'ancrage (ou effet « Press-Fit ») au niveau d'une zone du fémur préalablement préparée est de même dimension pour les deux tiges de la gamme intermédiaire. Dans l'exemple pris ici, la zone conique a un diamètre proximal de 20 mm (c'est ce diamètre qui sert à nominer l'implant) et une hauteur de 30 mm. La pente conique est en moyenne de 6 à 9 degrés, mais elle peut varier entre 2 et 12 degrés.

Cette zone conique Z1 ou Z2 est surtout caractérisée par le fait qu'elle ne se situe pas au même niveau sur les deux tiges de la gamme intermédiaire. C'est ainsi que la zone Z2 de l'une des tiges se situe dans une position plus basse d'environ 10 mm par rapport à la zone Z1 de l'autre tige, et il en serait de même pour une zone Z3, si elle existait, par rapport à la zone Z2. La progression de la zone d'ancrage peut être prédéterminée avec un incrément de 1 cm par exemple.

Un tel dispositif permet de ne pas faire varier la longueur de la tige en fonction du diamètre de la zone utile ou nécessaire pour assurer la stabilité primaire de l'implant, et il est donc possible de réaliser une gamme d'implants composée de tiges qui sont toujours d'une longueur identique. Surtout, ces tiges peuvent être les plus courtes possibles, quel que soit le positionnement de la zone « Press-Fit » dans le fût fémoral, puisque l'on peut choisir avec précision la zone de stabilité, et que l'on sait par ailleurs qu'un contact os-implant sur une hauteur de 3 cm est suffisant pour assurer la stabilité par effet « Press-Fit ». Dans ces conditions, en prenant pour référence le centre de rotation col moyen et l'extrémité distale de l'implant, la longueur de chaque implant est d'environ 115 mm, ce qui est nettement inférieur à la longueur des tiges sans ciment habituellement implantées.

Ce dispositif procure également beaucoup de souplesse pour régler d'une façon précise la longueur du membre inférieur, ce qui est souvent délicat avec un implant sans ciment dont la qualité de la stabilité dépend aussi du degré d'enfoncement de l'implant. Pour illustrer cette possibilité, prenons l'exemple d'une tige de diamètre 20 mm dont la stabilité est assurée avec la zone conique en position Z1 et qui s'avère être, dans ce cas, une position trop basse d'environ 10 mm par rapport au sommet du grand trochanter. Deux possibilités s'offrent alors à l'opérateur : soit il fait le choix d'une tige de même taille, mais avec cette fois une zone d'ancrage en position Z2 ; soit il fait le choix d'un implant de taille supérieure. Dans ce cas, en supposant une progression du diamètre de 2 mm en 2 mm, il est possible de remplacer cet implant par un implant de taille 22 mm, avec une zone conique en position Z1. Ces deux implants occupent très sensiblement le même espace endomédullaire en positionnant le deuxième implant en position plus haute de 10 mm, ce qui correspond à la différence de longueur à corriger, et cette manoeuvre permet de restituer la bonne longueur du membre inférieur sans augmenter la longueur de l'implant.

Le diamètre des tiges peut varier d'une façon millimétrique, mais la progression peut également se faire de 2 mm en 2 mm. Bien entendu, et comme pour la longueur et les autres caractéristiques chiffrées, les dimensions données n'ont qu'une valeur d'exemple et celles-ci peuvent être différentes.

Selon une autre caractéristique, ces tiges comprennent des ailettes de blocage complémentaires. Par ailleurs, le mode de blocage est conique de face, et celui-ci se fait surtout dans le plan latéro-médial.

L'invention concerne également un dispositif de préparation d'un fût fémoral en vue de l'implantation d'une tige prothétique telle que décrite ci-dessus.

Cette préparation s'effectue à l'aide de râpes distales bibloc surmontées d'un manche amovible, puis ensuite d'une partie proximale d'essai ayant la même forme que l'implant définitif, ces râpes présentant une connexion permettant de les transformer, par l'intermédiaire de cols d'essai, en prothèse d'essai.

Ce dispositif comprend d'une part une râpe modulable comprenant une pièce distale, fortement conique de taille et de forme unique, et dont au moins deux pièces proximales de formes sensiblement différentes et de hauteurs croissantes, pouvant être solidarisées de la pièce distale par des moyens de liaison amovibles, selon un choix déterminé en fonction du morphotype, et du degré d'enfoncement de la pièce distale, et d'autre part un élément intermédiaire neutre formant une jauge proximale destinée à la mise en place préalable sur la partie distale et à la mesure du degré d'enfoncement de la pièce distale, selon une graduation relativement au sommet du grand trochanter du fémur lorsque la pièce distale adopte sa position enfoncée recherchée, indiquant la bonne longueur du fémur à reconstituer et conséquemment la taille de la pièce proximale de la râpe à choisir et à disposer en lieu et place de l'élément intermédiaire en vue de la préparation dudit fût fémoral.

## Revendications

1. Gamme de prothèses fémorales de première intention, à implanter sans ciment dans un fût fémoral, dans une zone dite de « Press-Fit » se situant dans celui-ci à une hauteur variable selon le morphotype et l'importance des défauts osseux, **caractérisée en ce qu'**elle comprend, pour chaque diamètre, au moins deux tiges fémorales coniques (1, 2), de longueurs identiques (L1, L2), d'angles cervico-diaphysaire également identiques, et dont la zone (Z1, Z2) qui viendra au contact de la zone « Press-Fit » du fût fémoral préalablement préparé et assurera le blocage de l'implant est de dimensions identiques pour les deux tiges, mais n'est pas située au même niveau, la zone de blocage de l'une des tiges (Z2) étant relativement plus basse que la zone de blocage de l'autre tige (Z1), de manière que ladite tige retenue (1 ou 2) soit toujours la plus courte possible quel que soit le positionnement de la zone « Press-Fit » dans le fût fémoral, et de manière à assurer dans tous les cas la bonne longueur du membre inférieur.

2. Gamme de prothèses selon la revendication 1, **caractérisée en ce que** la conicité de la zone de blocage (Z1, Z2) des tiges (1, 2) est comprise entre 2 et 12 degrés selon leur diamètre.

3. Gamme de prothèses selon l'une des revendications 1 ou 2, **caractérisée en ce que** les tiges (1, 2) comprennent des ailettes de blocage complémentaires.

4. Gamme de prothèses selon l'une des revendications 1 à 3, **caractérisée en ce que** les diamètres des tiges (1, 2) varient de façon millimétrique.

5. Dispositif de préparation d'un fût fémoral en vue de l'implantation d'une tige prothétique appartenant à une gamme de prothèses (1, 2, 3) selon l'une des revendications 1 à 4, **caractérisé en ce qu'**il comprend des râpes distales bibloc surmontées d'un manche amovible, puis ensuite d'une partie proximale d'essai ayant la même forme que l'implant définitif, ces râpes présentant une connexion permettant de les transformer, par l'intermédiaire de cols d'essai, en prothèse d'essai.

6. Dispositif selon la revendication 5, **caractérisé en ce qu'**il comprend d'une part une râpe modulable comprenant une pièce distale, fortement conique de taille et de forme unique, et au moins deux pièces proximales de formes sensiblement différentes et de hauteurs croissantes, pouvant être solidarisées de la pièce distale par des moyens de liaison amovibles, selon un choix déterminé en fonction du morphotype, et du degré d'enfoncement de la pièce distale, et d'autre part un élément intermédiaire neutre formant une jauge proximale destinée à la mise en place préalable sur la partie distale et à la mesure du degré d'enfoncement de la pièce distale, selon une graduation relativement au sommet du grand trochanter du fémur lorsque la pièce distale adopte sa position enfoncée recherchée, indiquant la bonne longueur du fémur à reconstituer et conséquemment la taille de la pièce proximale de la râpe à choisir et à disposer en lieu et place de l'élément intermédiaire en vue de la préparation dudit fût fémoral.

## Patentansprüche

1. Femurprothesenset für den Ersteinsatz zum zementlosen Implantieren in einen Femurschacht in einem sogenannten "Press-Fit"-Bereich, der sich in diesem in einer je nach Morphotyp und Größe der Knochenfehler variablen Höhe befindet, **dadurch gekennzeichnet, dass** es für jeden Durchmesser mindesten zwei konische Femurschäfte (1, 2) identischer Längen (L1, L2) und ebenfalls identischer Zerviko-Diaphysen-Winkel umfasst, deren Zone (Z1, Z2), die mit der "Press-Fit"-Zone des zuvor vorbereiteten Femurschachts in Kontakt kommt und die Blockade des Implantats sichert, für die beiden Schäfte identische Abmessungen hat, sich aber nicht auf demselben Niveau befindet, wobei die Blockadezone einer der Schäfte (Z2) relativ niedriger ist als die Blockadezone des anderen Schafts (Z1), so dass der gehaltene Schaft (1 oder 2) unabhängig von der Positionierung der "Press-Fit"-Zone im Femurschacht immer so kurz wie möglich ist und in allen Fällen die richtige Länge der unteren Gliedmaße sicherstellt.

2. Prothesenset nach Anspruch 1, **dadurch gekennzeichnet, dass** die Konizität der Blockadezone (Z1, Z2) der Schäfte (1, 2) gemäß ihrem Durchmesser zwischen 2 und 12 Grad inklusive ist.

3. Prothesenset nach einem der Ansprüche 1 oder 2, **dadurch gekennzeichnet, dass** die Schäfte (1, 2) komplementäre Blockierflügel umfassen.

4. Prothesenset nach einem der Ansprüche 1 bis 3, **dadurch gekennzeichnet, dass** die Durchmesser der Schäfte (1, 2) millimeterweise variieren.

5. Vorrichtung zur Vorbereitung eines Femurschachts zwecks Implantation eines prothetischen Schafts, der zu einem Prothesenset (1, 2, 3) nach einem der Ansprüche 1 bis 4 gehört, **dadurch gekennzeichnet, dass** sie distale Zweiblockraspeln mit einem lösbaren Handgriff und danach einem proximalen Versuchsteil mit derselben Form wie das endgültige Implantat umfasst, wobei diese Raspeln eine Verbindung aufweisen, die erlaubt, sie mit Hilfe von Versuchshälsen in eine Versuchsprothese umzuwandeln.

6. Vorrichtung nach Anspruch 5, **dadurch gekennzeichnet, dass** sie einerseits eine modulierbare Raspel umfasst, die ein größenmäßig stark konisches, einzig geformtes distales Teil und mindestens zwei proximale Teile mit deutlich unterschiedlichen Formen und zunehmenden Höhen umfasst, die mit dem distalen Teil mit lösbaren Verbindungsmitteln gemäß einer bestimmten Wahl in Abhängigkeit vom Morphotyp und vom Eindringgrad des distalen Teils verbindbar sind, und andererseits ein neutrales Übergangselement, das eine proximale Latte bildet, die zur vorherigen Platzierung auf dem distalen Teil und zum Messen des Endringgrads des distalen Teils gemäß einer Skalierung relativ zur Spitze des Trochanter major des Oberschenkelknochens bestimmt ist, wenn das distale Teil seine angestrebte eingedrungene Position einnimmt, wobei die richtige Länge des wiederherzustellenden Oberschenkelknochens angezeigt wird und folglich die Größe des proximalen Teils der Raspel, das auszuwählen und anstelle des Übergangselements zwecks Vorbereitung des Femurschachts anzuordnen ist.

## Claims

1. A range of first-line femoral prostheses, to be implanted without any cement into a femoral shaft, in a so-called "press-fit" area located in the latter at a variable height according to the morphotype and to the extent of bone defects, **characterized in that** it comprises, for each diameter, at least two conical femoral rods (1, 2), with identical lengths (L1, L2), with also identical neck/shaft angles, and for which the area (Z1, Z2) which will come into contact with the "press-fit" area of the femoral shaft prepared beforehand and will ensure blocking of the implant, is of identical dimensions for both rods, but is not located at the same level, the blocking area of one of the rods (Z2) being a relatively lower than the blocking area of the other rod (Z1), so that said retained rod (1 or 2) is always the shortest as possible regardless of the positioning of the "press-fit" area in the femoral shaft, and so as to ensure in every case the proper length of the lower limb.

2. The range of prostheses according to claim 1, **characterized in that** the conicity of the blocking area (Z1, Z2) of the rods (1, 2) is comprised between 2 and 12 degrees depending on their diameter.

3. The range of prostheses according to one of claims 1 or 2, **characterized in that** the rods (1, 2) comprise additional blocking fins.

4. The range of prostheses according to one of claims 1 to 3, **characterized in that** the diameters of the rods (1, 2) vary millimetrically.

5. A device for preparing a femoral shaft with view to implementing a prosthetic rod belonging to a range of prostheses (1, 2, 3) according to one of claims 1 to 4, **characterized in that** it comprises biblock distal rasps surmounted with a movable sleeve, and then subsequently a test proximal portion having the same shape as the definitive implant, these rasps having a connection allowing them to be transformed via test necks, into a test prosthesis.

6. The device according to claim 5, **characterized in that** it comprises a modular rasp comprising a distal part, highly conical in size and with a unique shape, and at least two proximal parts of substantially different shapes and with increasing heights, which may be secured to the distal part by removable connection means according to a selection determined according to the morphotype, and to the degree of penetration of the distal part on the one hand, and a neutral intermediate element forming a proximal gauge intended for setting into place beforehand on the distal portion and for measuring the penetration degree of the distal part, according to a graduation relatively to the top of the greater trochanter of the femur when the distal part adopts its sought penetration position, on the other hand, indicating the proper length of the femur to be restored and consequently the size of the proximal part of the rasp to be selected and positioned instead and in place of the intermediate element with view to the preparation of said femoral shaft.
